(19) Europäisches Patentamt / European Patent Office / Office européen des brevets

(11) **EP 4 455 113 A1**

(12) **EUROPEAN PATENT APPLICATION**
published in accordance with Art. 153(4) EPC

(43) Date of publication:
**30.10.2024 Bulletin 2024/44**

(51) International Patent Classification (IPC):
*C07C 1/04* (2006.01)        *C07C 1/24* (2006.01)
*C07C 15/08* (2006.01)       *C07C 29/151* (2006.01)
*C07C 31/04* (2006.01)       *C07B 61/00* (2006.01)

(21) Application number: 22911330.3

(22) Date of filing: **22.12.2022**

(52) Cooperative Patent Classification (CPC):
**C07C 1/04; C07C 1/24; C07C 15/08; C07C 29/151;
C07C 31/04;** C07B 61/00

(86) International application number:
**PCT/JP2022/047306**

(87) International publication number:
**WO 2023/120628 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.12.2021 JP 2021209637**

(71) Applicant: **Chiyoda Corporation
Kanagawa 220-8765 (JP)**

(72) Inventor: **HIROHATA, Osamu
Yokohama-shi, Kanagawa 220-8765 (JP)**

(74) Representative: **Wächtershäuser & Hartz
Patentanwaltspartnerschaft mbB
Weinstraße 8
80333 München (DE)**

(54) **METHOD FOR MANUFACTURING PARAXYLENE**

(57)     An effective method for adjusting the hydrogen/carbon ratio of synthesis gas, which is a raw material for efficiently directly producing paraxylene, is provided. A method for producing paraxylene from synthesis gas obtained by gasifying carbon-containing raw materials, comprising a mixing step of obtaining a raw material mixed gas by adding hydrogen to the synthesis gas; a reaction step of obtaining a mixed gas product containing paraxylene by introducing the raw material mixed gas into a reactor and bringing it into contact with a paraxylene synthesis catalyst under heating and pressurization; a separation step of separating the mixed gas product into a gas phase composed of non-condensable components, an oil phase containing paraxylene, and an aqueous phase containing water-soluble components by cooling the mixed gas product to condense high-boiling point components; and a purification step of purifying the oil phase to obtain paraxylene,
wherein the mixing step adds hydrogen so that the obtained raw material mixed gas has a composition in which an R value defined by the following formula (1)

$$R = \frac{N_{H2} - N_{CO2}}{N_{CO} + N_{CO2}} \qquad (1)$$

falls within a range of 0.7 or more and 2.1 or less.

EP 4 455 113 A1

## FIG. 3

RELATIONSHIP BETWEEN R VALUE AND PX PRODUCTION AMOUNT

FEED SYNTHESIS GAS AMOUNT + RENEWABLE ENERGY H2 AMOUNT + PSA H2 AMOUNT = CONSTANT AT 1419 kgmol/h

CONSTANT RECYCLE AMOUNT AND REACTOR SIZE

## Description

### Technical Field

The present invention relates to a method for directly producing paraxylene from synthesis gas obtained by gasifying carbon-containing raw materials, especially raw materials containing solid carbon-containing raw materials such as waste plastics and other wastes, biomass, coal, and petroleum coke.

### Background Art

Paraxylene, which is useful as a raw material for polyester fibers and polyethylene terephthalate (PET) resins, has conventionally been produced by reforming naphtha in a petrochemical complex, but this method requires fossil (petroleum) resources and emits a large amount of carbon dioxide during the manufacturing process.

Therefore, as a method for producing paraxylene without using fossil resources, a method using synthesis gas containing carbon monoxide and hydrogen as a raw material has already been proposed (Non-Patent Literature 1, Patent Literature 1). In this method, the synthesis gas is converted to methanol by a catalyst having a $ZnCr_2O_4$ spinel structure, etc., and then the methanol is converted to aromatic compounds containing paraxylene by a catalyst such as H-ZSM-5 zeolite (proton-type ZSM-5 zeolite) whose outer surface is coated with silicalite-1. Additionally, by using these catalysts in a mixture, paraxylene can be synthesized from synthesis gas containing carbon monoxide and hydrogen in a one-step reaction operation. In addition, a method has also been proposed in which carbon dioxide is used instead of carbon monoxide, and paraxylene is synthesized in one step using this and hydrogen as raw materials (Patent Literature 2). The method of Patent Literature 2 uses a chromium oxide catalyst as a methanol synthesis catalyst and a H-ZSM-5 zeolite coated with silicalite-1 as a paraxylene synthesis catalyst to improve the production efficiency of paraxylene; by using the methanol synthesis catalyst and the paraxylene synthesis catalyst in a mixture, paraxylene is synthesized from synthesis gas containing carbon dioxide and hydrogen in a one-step reaction operation.

On a related note, synthesis gas, which is a raw material for paraxylene synthesis, is produced from various raw materials containing carbon, but the composition of the synthesis gas obtained varies depending on the raw material and production conditions. For example, the composition of synthesis gas obtained by reforming natural gas (the content ratio of hydrogen to carbon monoxide and carbon dioxide) varies depending on the addition ratio of steam and carbon dioxide that react with the natural gas. In addition, since synthesis gas obtained by gasifying solid carbon-containing raw materials such as waste plastics, biomass, coal, and petroleum coke has a smaller molar ratio of hydrogen to carbon monoxide and carbon dioxide (hydrogen/carbon ratio) than synthesis gas obtained by reforming natural gas, the hydrogen/carbon ratio may be adjusted before using it as a process raw material. For example, Patent Literature 3 describes that hydrogen obtained by electrolysis of water is mixed with synthesis gas, or a part of the flow of synthesis gas is branched and carbon monoxide therein is converted to hydrogen by a water gas shift reaction, and then the branched flow is merged again into the original synthesis gas flow, so that the hydrogen/carbon ratio is adjusted to the optimum ratio for synthesizing other chemical products and fuels. Further, Patent Literature 4 describes that liquid hydrocarbon fuel is efficiently synthesized from synthesis gas obtained by gasifying coal by partially removing carbon dioxide from the synthesis gas (and adjusting the hydrogen/carbon ratio).

### Citation List

#### Patent Literature

PTL 1: Published Japanese Translation of PCT International Application No. 2020-535966
PTL 2: Japanese Patent Application Laid-Open No. 2019-205969
PTL 3: Japanese Patent Application Laid-Open No. 2010-70763
PTL 4: U.S. Patent Application Publication No. 2008/0098654

#### Non Patent Literature

NPL 1: Peipei Zhang et al., Chemical Science, The Royal Society of Chemistry, October 2017, Vol. 8, pp. 7941-7946

## Summary of Invention

### Technical Problem

When paraxylene is to be synthesized in one step from synthesis gas by the method of Non-Patent Literature 1 or Patent Literature 1 or Patent Literature 2, especially when using synthesis gas produced by gasifying solid carbon-containing raw materials, the content of hydrogen relative to carbon monoxide and carbon dioxide is insufficient, and the conversion rate of carbon monoxide and carbon dioxide cannot be increased, so that efficient paraxylene synthesis cannot be achieved. In this regard, Patent Literature 3 describes increasing the proportion of hydrogen in the synthesis gas by adding hydrogen obtained by electrolysis of water or by a water gas shift reaction, but Patent Literature 3 is mainly focused on the case of producing synthetic fuels, ammonia, and ethanol from synthesis gas, and is not directed to the synthesis of paraxylene, and the specific optimum ratio for each product has not been studied. In addition, in the water gas shift reaction, while carbon monoxide is consumed, an equimolar amount of carbon dioxide is produced at the same time, and since carbon dioxide requires twice as much hydrogen as carbon monoxide to be deoxidized, it is not suitable as a method for increasing the molar ratio of hydrogen to carbon monoxide and carbon dioxide (hydrogen/carbon ratio) so that it is optimal for fuel and chemical synthesis. Further, Patent Literature 4 describes that the hydrogen/carbon ratio is adjusted to the optimum ratio for the synthesis of paraxylene by reducing the proportion of carbon in the synthesis gas by removing carbon dioxide from the synthesis gas, but since the use of the removed carbon dioxide is not considered, there is a problem in terms of reduction of carbon dioxide emissions and effective use of carbon resources, and it is mainly directed to the production of synthetic fuels (liquid hydrocarbon fuels) from synthesis gas, and is not directed to the direct synthesis of paraxylene from synthesis gas. As described above, an effective method for adjusting the hydrogen/carbon ratio of synthesis gas, which is a raw material for efficiently directly producing paraxylene, has not yet been found.

### Solution to Problem

The present invention provides a method for producing paraxylene from synthesis gas obtained by gasifying carbon-containing raw materials, comprising a mixing step of obtaining a raw material mixed gas by adding hydrogen to the synthesis gas; a reaction step of obtaining a mixed gas product containing paraxylene by introducing the raw material mixed gas into a reactor and bringing it into contact with a paraxylene synthesis catalyst under heating and pressurization; a separation step of separating the mixed gas product into a gas phase composed of non-condensable components, an oil phase containing paraxylene, and an aqueous phase containing water-soluble components by cooling the mixed gas product to condense high-boiling point components; and a purification step of purifying the oil phase to obtain paraxylene, wherein the mixing step adds hydrogen so that the obtained raw material mixed gas has a composition in which an R value defined by the following formula (1) [Math. 1]

$$R = \frac{N_{H2} - N_{CO2}}{N_{CO} + N_{CO2}} \qquad (1)$$

(where $N_{H2}$ represents a molar fraction of hydrogen, $N_{CO}$ represents a molar fraction of carbon monoxide, and $N_{CO2}$ represents a molar fraction of carbon dioxide) falls within a range of 0.7 or more and 2.1 or less, thereby solving the above problem.

### Advantageous Effects of Invention

According to the method of the present invention, since a criterion is provided for adjusting to a suitable molar ratio of hydrogen to carbon monoxide and carbon dioxide (hydrogen/carbon ratio) for paraxylene synthesis by adding hydrogen to synthesis gas having a low hydrogen content ratio, it is possible to prevent excessive addition of hydrogen and suppress the amount of hydrogen added to the minimum necessary; at the same time, it is possible to efficiently add hydrogen by appropriately selecting means for supplying the necessary hydrogen, and the optimization of the paraxylene production process using synthesis gas produced by gasifying solid carbon-containing raw materials can be achieved.

### Brief Description of Drawings

Fig. 1 shows an example of a process flow of the embodiment which implements the method of the present invention.

Fig. 2 shows an example of the purification step in the process flow of Fig. 1.

Fig. 3 shows the relationship between the R value and the paraxylene production amount.

## Description of Embodiments

In general, a mixed gas of carbon dioxide or carbon monoxide or both with hydrogen is called synthesis gas, and a product gas mixture containing paraxylene can be obtained by heating and pressurizing this synthesis gas and bringing it into contact with a paraxylene synthesis catalyst (reaction step). Then, by cooling the obtained product gas mixture to condense the high-boiling point components, it is separated into an aqueous phase containing water-soluble components, an oil phase containing paraxylene, and a gas phase containing unreacted gas (separation step). Since the oil phase obtained there is a mixture containing various aromatic components in addition to paraxylene, purified paraxylene can be obtained by separating the mixture into each aromatic component by separation means such as distillation (purification step). That is, the process for producing paraxylene from synthesis gas is roughly divided into a reaction step, a separation step, and a purification step. Hereinafter, preferred embodiments for carrying out the present invention will be described.

<Reaction Step>

The synthesis gas used as a raw material in the reaction step can be obtained by various methods. For example, the synthesis gas obtained by reforming natural gas whose main component is methane has a molar ratio of hydrogen to carbon monoxide or carbon dioxide (hydrogen/carbon ratio) of about 3:1 to 1:1, and is suitable for obtaining a mixture of various aromatic compounds through the production of methanol and dimethyl ether as it is. On the other hand, in the synthesis gas obtained by gasifying solid carbon-containing raw materials such as waste plastics and other wastes, biomass, coal, and petroleum coke, the hydrogen/carbon ratio is about 1:2 to 1:5, and the molar ratio of hydrogen contained is relatively small compared to the synthesis gas obtained by reforming natural gas. When such synthesis gas having a small molar ratio of hydrogen is used as a raw material as it is, it is expected that the overall reaction efficiency will decrease because the conversion rate of carbon monoxide and carbon dioxide to methanol, etc. is low.

Specifically, the following can be said. That is, when a mixed gas of carbon monoxide and hydrogen is reacted, it is considered that methanol and dimethyl ether are produced as shown in formula (1), and the methanol and dimethyl ether thus produced are converted to a mixture of various aromatic compounds via lower olefins as shown in formula (2).

$$2CO + 4H_2 \rightarrow 2CH_3OH \ (\Leftrightarrow CH_3OCH_3 + H_2O) \ (1)$$

$$CH_3OCH_3 \rightarrow C_2H_4, C_3H_6, \text{etc.} \rightarrow \text{Various aromatic compounds} \qquad (2)$$

In other words, stoichiometrically, carbon monoxide and hydrogen react in a molar ratio of 1:2. In this case, as a catalyst for promoting the methanol synthesis reaction of formula (1), a catalyst having a spinel structure composed of a composite oxide of zinc (or copper) and chromium can be preferably used. Further, as a catalyst for promoting the reaction of formula (2) to selectively synthesize paraxylene, H-ZSM-5 zeolite can be preferably used. H-ZSM-5 zeolite may be doped with zinc or the like. At this time, if the outer surface of the H-ZSM-5 zeolite is coated with a silicon-containing compound (preferably one having the same lattice structure as ZSM-5 zeolite and having no acid sites, such as silicalite-1), the proportion of paraxylene in the product mixture can be increased. In addition, if these catalysts are used in a mixture, the reaction of formula (1) and the reaction of formula (2) proceed continuously or in parallel, so that a product containing paraxylene can be produced in a single reactor.

On the other hand, when a mixed gas of carbon dioxide and hydrogen is reacted, the reaction to produce methanol (and dimethyl ether) proceeds as shown in formula (3).

$$CO_2 + 3H_2 \rightarrow CH_3OH + H_2O \qquad (3)$$

In other words, stoichiometrically, carbon dioxide and hydrogen react in a molar ratio of 1:3. In this case, since water is produced as a by-product at the same time as methanol is produced, as described in Patent Literature 2, as a catalyst for promoting the reaction of formula (3), a catalyst composed of chromium oxide (not containing zinc or copper) is used instead of the above-mentioned catalyst composed of a composite oxide of zinc (or copper) and chromium; as a catalyst for promoting the reaction of formula (2), a proton-type H-ZSM-5 without zinc doping is used, which can increase the yield of paraxylene. At this time, as in the case of reacting carbon monoxide and hydrogen, if the outer surface of the H-ZSM-5 zeolite is coated with a silicon-containing compound (preferably one having the same lattice structure as ZSM-5 zeolite and having no acid sites, such as silicalite-1), the proportion of paraxylene in the product mixture can be increased. In addition, if these catalysts are used in a mixture, the reaction of formula (3) and the reaction of formula (2) proceed continuously or in parallel, so that a product containing paraxylene can be produced in a single reactor.

That is, in the reaction step, a catalyst containing at least one metal oxide appropriately selected from chromium, zinc, and copper according to the ratio of carbon dioxide to carbon monoxide and the content of other components in the synthesis gas (strictly speaking, the raw material gas mixture at the reactor inlet) used as a raw material; and a catalyst containing H-ZSM-5 zeolite, which is appropriately doped with zinc or the like, coated with a silicon-containing compound such as silicalite-1 may be used in combination. In this case, since the synthesis gas obtained by reforming natural gas has a suitable hydrogen/carbon ratio of 3:1 to 2:1, the methanol synthesis reaction proceeds efficiently even as it is, whereas the synthesis gas obtained by gasifying solid carbon-containing raw materials has a hydrogen/carbon ratio of 1:2 to 1:5, so that the hydrogen required for the reaction will be insufficient as it is. The reason why the carbon/hydrogen molar ratio in the synthesis gas obtained by gasifying solid carbon-containing raw materials becomes small is, firstly, that the proportion of hydrogen atoms in the raw materials is smaller than the proportion of hydrogen atoms in natural gas (methane); in addition to that, gasification of solid raw materials requires a large amount of thermal energy for the reaction and is not suitable for indirect heating via a heat transfer wall, so that instead of steam, oxygen is mainly supplied to partially oxidize carbon and hydrogen in the raw materials to obtain the necessary thermal energy. Specifically, the following reactions occur in a complex manner in the gasification step.

$C_aH_bO_c \rightarrow CO + CO_2 + H_2O +$ hydrocarbons (thermal decomposition)
$C + 1/2O_2 \rightarrow CO$ (partial oxidation)
$C + H_2O \rightarrow CO + H_2$ (water gasification)
$CO + H_2O \rightarrow CO_2 + H_2$ (shift)
$C + CO_2 \rightarrow 2CO$ (generator gasification)
$C + 2H_2 \rightarrow CH_4$ (hydrogenation gasification)

Among the reactions listed above, partial oxidation, shift, and hydrogenation gasification are exothermic reactions, while thermal decomposition, water gasification, and generator gasification are endothermic reactions, so that the thermal energy required for gasification is mainly supplied by partial oxidation. This is the reason why oxygen supply is necessary.

As mentioned above, since the synthesis gas obtained by gasifying solid carbon-containing raw materials has a hydrogen/carbon ratio considerably smaller than the stoichiometric ratio of the starting reaction, which is the methanol synthesis reaction, the reaction efficiency (conversion rate of carbon monoxide and carbon dioxide to methanol) is expected to decrease if it is used as it is, so that it is necessary to supplement hydrogen. The present invention has been made by studying how the reaction efficiency changes depending on the hydrogen/carbon ratio of the raw material synthesis gas.

That is, the following reaction model is assumed, in which carbon monoxide and carbon dioxide in the synthesis gas react with hydrogen to produce paraxylene.

$$8CO + 13H_2 \rightarrow C_8H_{10} + 8H_2O$$

$$8CO_2 + 21H_2 \rightarrow C_8H_{10} + 16H_2O$$

In other words, 13 moles of hydrogen are required to convert 8 moles of carbon monoxide to 1 mole of paraxylene, and 21 moles of hydrogen are required to convert 8 moles of carbon dioxide to 1 mole of paraxylene, so that if there are $(13/8)N_{CO}$ moles of hydrogen for $N_{CO}$ moles of carbon monoxide and $(21/8)N_{CO2}$ moles of hydrogen for $N_{CO2}$ moles of carbon dioxide, all carbon monoxide and carbon dioxide can be converted to paraxylene. Therefore, if the molar amount of hydrogen is $N_{H2}$,

$$N_{H2} = (13/8)N_{CO} + (21/8)N_{CO2}$$

is the stoichiometrically optimum quantitative ratio for paraxylene synthesis. By transforming this equation, we get

$$8N_{H2} = 13N_{CO} + 21N_{CO2}$$

$$8N_{H2} = 13(N_{CO} + N_{CO2}) + 8N_{CO2}$$

$$8(N_{H2} - N_{CO2}) = 13(N_{CO} + N_{CO2})$$

$$(N_{H2} - N_{CO2})/(N_{CO} + N_{CO2}) = 13/8$$

Therefore, when the R value defined by formula (1) [Math. 2]

$$R = \frac{N_{H2} - N_{CO2}}{N_{CO} + N_{CO2}} \tag{1}$$

(where $N_{H2}$ represents a molar fraction of hydrogen, $N_{CO}$ represents a molar fraction of carbon monoxide, and $N_{CO2}$ represents a molar fraction of carbon dioxide) is 13/8 = 1.625, the quantitative ratio of hydrogen to carbon monoxide and carbon dioxide is stoichiometrically optimum for paraxylene synthesis. Additionally, the present inventors have confirmed that the paraxylene production amount is maximized if the R value thus defined is 0.7 or more and 2.1 or less, preferably 0.9 to 2.0, for the synthesis gas used as a raw material, as specifically shown in Examples below.

That is, the present invention is characterized in that the raw material mixed gas supplied to the reaction step is a synthesis gas adjusted by mixing hydrogen with synthesis gas produced by gasifying carbon-containing raw materials so that the R value defined by the above formula (1) falls within a range of 0.7 or more and 2.1 or less. In a preferred embodiment of the present invention, the mixed gas product produced in the reaction step is cooled in the separation step to form an oil phase and an aqueous phase from condensed components, and non-condensable components that form a gas phase are recycled to the inlet side of the reactor and supplied to the reaction step again. The raw material mixed gas to which hydrogen has been added to the synthesis gas is supplied by merging into this recycle loop. In this case, the R value defined above is a value immediately before the raw material mixed gas merges into the recycle loop (recycle loop inlet). That is, the R value must be defined for the raw material mixed gas newly supplied to the reaction system, not including the non-condensable components recycled to the reactor.

The synthesis gas constituting the raw material mixed gas by adding hydrogen is obtained by gasifying carbon-containing raw materials. Such carbon-containing raw materials are assumed to be solid carbon-containing raw materials such as waste plastics, paper scraps and other wastes, wood chips and other biomass, coal, and petroleum coke, but are not necessarily limited thereto. That is, in the present invention, the synthesis gas constituting the raw material mixed gas by adding hydrogen is not necessarily obtained by gasifying solid carbon-containing raw materials, as long as it is a synthesis gas having a small hydrogen/carbon ratio, which is not suitable for paraxylene synthesis without adding hydrogen (the R value is less than 0.7). For example, off-gas discharged from a blast furnace in a steelworks, off-gas separated in a hydrogen production apparatus, synthesis gas produced by co-electrolysis of water and carbon dioxide, synthesis gas produced by a reverse shift reaction of hydrogen and carbon dioxide, and the like may be used.

The hydrogen added to the synthesis gas to constitute the raw material mixed gas is not particularly limited, but in the preferred embodiment of the present invention described above, the non-condensable components that form the gas phase in the separation step and are recycled to the inlet side of the reactor contain unreacted hydrogen, and a part thereof is extracted from the recycle loop as purge gas, so that hydrogen recovered from the purge gas by a hydrogen separator can be used. However, since the hydrogen recovered from the purge gas generally alone does not cover the total amount of hydrogen added to the synthesis gas, the shortage needs to be covered by, for example, hydrogen generated by electrolyzing water. The electric power required for the electrolysis of water in this case is preferably electric power generated by renewable energy such as sunlight, wind power, hydropower, geothermal energy, and biomass.

The form of the reactor for obtaining a product gas mixture containing paraxylene from the raw material mixed gas is not particularly limited as long as it allows gas-solid contact operation between the raw material mixed gas (gas) and the reaction catalyst (solid) and can maintain desired temperature and pressure (packed bed, moving bed, fluidized bed, etc.), but a packed bed is preferable in terms of good contact efficiency, less channeling, and less mechanical damage to catalyst particles. The amount of catalyst charged and the gas flow rate can be set appropriately, but in the case of a packed bed type, it is preferable to set the amount of catalyst charged and the gas flow rate so that the space velocity (SV) is about 100 to 10000/hr on an empty tower basis. The reaction temperature is preferably set to about 250°C to 600°C, and the reaction pressure is preferably set to about 1 to 10 MPaG.

<Separation Step>

The product gas mixture containing paraxylene obtained in the reaction step is cooled in the subsequent separation step to condense the high-boiling point components containing paraxylene. The liquid phase is further divided into an aqueous phase containing water-soluble components such as water and alcohol generated in the reaction and an oil phase containing aromatic components, etc. (including paraxylene) that do not mix with water. That is, since the liquid phase is separated into an aqueous phase forming a lower layer, an oil phase forming a middle layer, and a gas phase forming an upper layer in order from the bottom side of the gas-liquid separator (gas-liquid-liquid separator), the fluid of each phase may be extracted to the outside of the apparatus from the position where each layer is formed. Alternatively, after cooling the product gas mixture, the obtained gas-liquid mixture may be first separated into a gas phase and a liquid phase, and then the liquid phase may be separated into an oil phase and an aqueous phase by a separation method utilizing the difference in specific gravity, such as centrifugation or sedimentation separation.

Since the gas phase extracted from the gas-liquid separator contains unreacted gases such as carbon dioxide, carbon monoxide, and hydrogen, it is returned to the inlet side of the heater, which is located upstream of the reactor, and circulated to the reactor. However, the gas phase contains lower alkanes having 1 to 4 carbon atoms (mainly methane) as by-products in addition to these unreacted gases, and since these lower alkanes hardly contribute to the paraxylene synthesis reaction in the reactor, these lower alkanes gradually accumulate in the gas in the circulation path. Therefore, it is necessary to purge a part of the gas in the circulation path to the outside. If about 1 to 20% by volume of the total circulation amount is purged, the lower alkane concentration in the circulation path can be maintained at less than 40% by volume.

The purged gas can be used as fuel gas because it contains carbon monoxide and hydrogen, and lower alkanes. However, as for the hydrogen contained in this purge gas, as described above, it is preferable to separate and recover it by membrane separation, adsorption separation (Pressure Swing Adsorption, etc.), etc., and use it for constituting the raw material mixed gas by adding it to the synthesis gas having a small hydrogen/carbon ratio (R value is less than 0.7).

The raw material mixed gas is heated on the inlet side of the reactor, and the product gas mixture is cooled on the outlet side of the reactor; it is preferable to use the heat recovered by cooling the product gas mixture for heating the raw material mixed gas, because energy required for heating and cooling can be saved. Further, when sufficient cooling of the product gas mixture cannot be expected by only heat exchange, the product gas mixture whose temperature has been lowered by the heat exchange operation to a certain degree may be further cooled.

<Purification Step>

Since the oil phase extracted from the gas-liquid separator contains other aromatic compounds such as benzene, toluene, ortho-xylene, meta-xylene, ethylbenzene, and trimethylbenzene in addition to the target compound paraxylene, these are separated as necessary. For this purpose, it is preferable to first subject the oil phase to a distillation operation to separate benzene and toluene having a boiling point lower than xylenes (ortho-xylene, meta-xylene, paraxylene, ethylbenzene) as low-boiling point components, and trimethylbenzene and the like having a boiling point higher than xylenes as high-boiling point components. On the other hand, since the boiling points of ortho-xylene, meta-xylene, and ethylbenzene are close to that of paraxylene, it is inefficient to separate them only by distillation. Therefore, it is preferable to obtain xylenes as a mixture thereof, and then adsorb and separate paraxylene from this mixture using zeolite.

Since zeolite has pores having a molecular size of paraxylene, it adsorbs paraxylene well, but hardly adsorbs ortho-xylene, meta-xylene, and ethylbenzene, functioning as a molecular sieve. That is, since components other than paraxylene (ortho-xylene, meta-xylene, ethylbenzene, and other impurities) are not adsorbed on the zeolite and pass through the adsorption tower, paraxylene can be concentrated and purified by repeating adsorption and desorption of this mixture using zeolite. Specifically, a high concentration of paraxylene can be obtained by flowing a xylene mixture through an adsorption tower packed with an adsorbent (zeolite) to adsorb only paraxylene, bringing a desorbent into contact with the adsorbent containing paraxylene to desorb paraxylene, and separating the mixture of desorbent and paraxylene in a distillation tower.

<Other Incidental Steps>

In order to increase the production of paraxylene, it is desirable to carry out isomerization treatment and disproportionation treatment as necessary. Ortho-xylene, meta-xylene, and ethylbenzene remaining after obtaining high-purity paraxylene in the purification step can be partially converted to paraxylene by isomerization treatment and then returned to the inlet side of the purification step. Specifically, the isomerization treatment may be carried out by heating a mixture of ortho-xylene, meta-xylene, and ethylbenzene after separating paraxylene and passing it through a reactor packed with a zeolite catalyst.

Further, toluene and trimethylbenzene separated by distillation can be partially converted to a xylene mixture containing paraxylene by disproportionation treatment and then returned to the inlet side of the purification step. Specifically, the disproportionation treatment may be carried out by heating a mixture containing toluene and trimethylbenzene and passing it through a reactor packed with a zeolite catalyst.

**Examples**

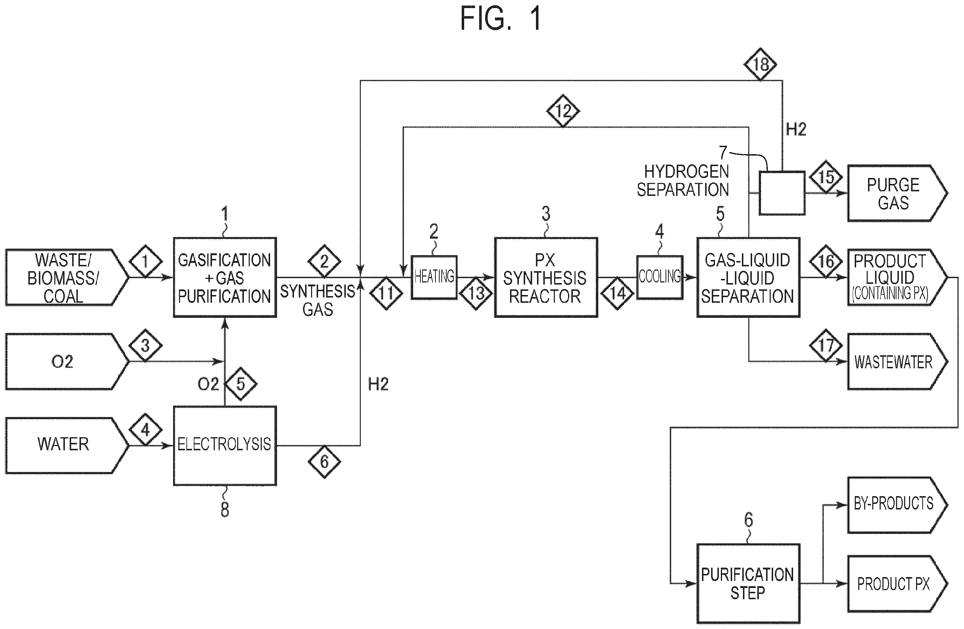

Fig. 1 shows an example of a process flow of an embodiment for carrying out the method of the present invention. In the method of the present invention, carbon-containing raw materials such as wastes, biomass, and coal are gasified in a gasifier 1 by partially combusting with oxygen introduced from outside, and the obtained gas mixture is purified as necessary to obtain synthesis gas mainly containing hydrogen, carbon monoxide, carbon dioxide, and the like. The synthesis gas thus obtained is heated by a temperature raising device 2 and then introduced into a reactor 3 for paraxylene synthesis. In the reactor 3, a catalyst containing at least one metal oxide selected from chromium, zinc, and copper and a catalyst containing ZSM-5 zeolite coated with silicalite-1 are mixed and filled to form a mixed catalyst layer, and the raw material gas mixture introduced into the reactor reacts by contacting with the mixed catalyst in the reactor under a high temperature and high pressure atmosphere of 250°C to 600°C and 1 to 10 MPaG to become a product gas mixture containing paraxylene (reaction step).

The obtained product gas mixture is cooled to near room temperature by a temperature lowering device 4 and introduced into a gas-liquid-liquid separator 5, where the condensed high-boiling point components are separated and form an aqueous phase containing water-soluble components (lower layer) and an oil phase containing paraxylene (middle layer), and a gas phase containing unreacted gas (upper layer) is formed on top of them (separation step).

The oil phase forming the middle layer is extracted from the gas-liquid-liquid separator 5 and then treated in a purification step 6 including distillation separation, adsorption separation, isomerization treatment, disproportionation treatment, etc., to obtain the target high-purity paraxylene (purification step). Details of the purification step will be described later.

Since the gas phase forming the upper layer contains unreacted gases such as hydrogen, carbon monoxide, and carbon dioxide, after being extracted from the gas-liquid-liquid separator 5, it is mixed with the raw material mixed gas flow on the inlet side of the temperature raising device 2 as circulating gas, heated again, and returned to the reactor 3. A part of the circulating gas is discharged out of the system as purge gas in order to prevent the accumulation of lower alkanes (methane, ethane, propane, etc.) contained in a small amount in the circulating gas in the system, and is effectively used as fuel gas for heat sources of nearby heating furnaces, etc. At this time, if hydrogen is separated from the purge gas by a hydrogen separator 7 which carries out separation operations such as pressure swing adsorption (PSA) and membrane separation using a hydrogen permeable membrane, the obtained hydrogen can be used for at least a part of the hydrogen to be added to the synthesis gas obtained in the gasifier 1.

The aqueous phase forming the lower layer in the gas-liquid-liquid separator 5 is fed to a wastewater treatment apparatus to remove water-soluble organic matter and the like, and after being treated, it is discharged out of the system.

In the present invention, in order to increase the hydrogen/carbon ratio of the synthesis gas obtained in the gasifier 1, hydrogen is added to the synthesis gas. As at least a part of the hydrogen to be added, as described above, the hydrogen separated from the purge gas by the hydrogen separator 7 can be used, but normally, the hydrogen separated from the purge gas alone does not cover the total amount of hydrogen to be added to the synthesis gas. Therefore, it is necessary to supply hydrogen from the outside, and as a means for that purpose, in Fig. 1, hydrogen is being generated by electrolysis of water in an electrolyzer 8. At this time, as hydrogen is generated from the cathode, oxygen is generated from the anode at the same time, so that the by-produced oxygen can be used for at least a part of the oxygen required for gasification. The electric power required for the electrolysis of water is preferably generated using renewable energy such as sunlight.

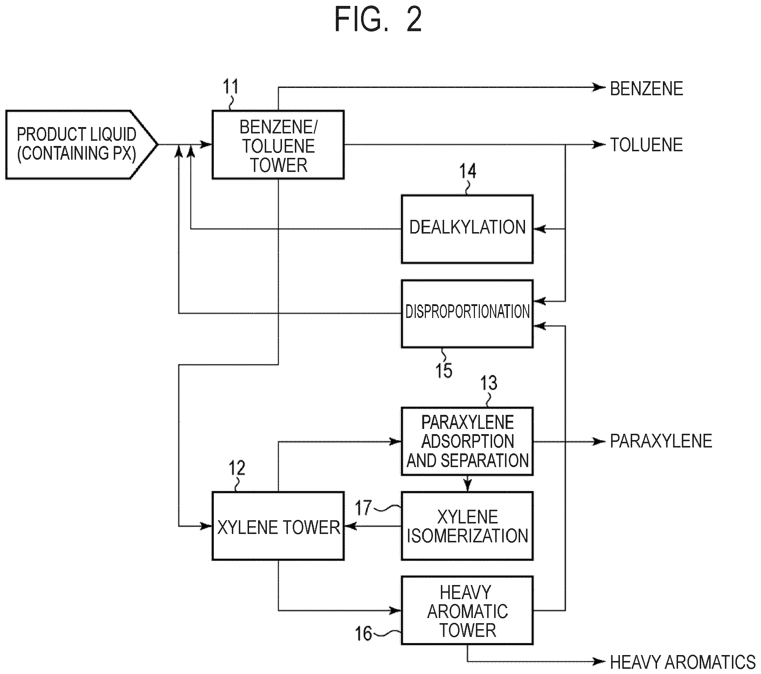

Fig. 2 shows an example of the purification step in the process flow of Fig. 1. Since the oil phase extracted from the gas-liquid-liquid separator 5 contains various aromatic compounds such as ortho-xylene, meta-xylene, benzene, toluene, ethylbenzene, and trimethylbenzene in addition to the target compound paraxylene of the present invention, these need to be separated and purified into individual compounds by operations such as distillation, adsorption, and extraction according to a conventional method. Specifically, the obtained mixture is first distilled in a benzene/toluene tower 11 to separate xylenes (ortho-xylene, meta-xylene, paraxylene) and ethylbenzene from low-boiling point components such as benzene and toluene.

The boiling points of xylenes (ortho-xylene, meta-xylene, paraxylene) and ethylbenzene remaining after separating and removing the low-boiling point components are close to each other, so that it is inefficient to separate them only by distillation. Therefore, it is possible to separate the mixture from heavy aromatics having 9 or more carbon atoms as a xylene fraction in a xylene distillation tower 12, obtain them together, and then separate and purify paraxylene by separating only paraxylene from the xylene fraction in a paraxylene adsorption apparatus 13 packed with zeolite. This is because zeolite has pores having a molecular size of paraxylene, and therefore adsorbs paraxylene well, while it hardly adsorbs ortho-xylene, meta-xylene, and ethylbenzene, functioning as a molecular sieve. That is, since components other than paraxylene (ortho-xylene, meta-xylene, ethylbenzene, and other impurities) are not adsorbed on the zeolite and pass through the paraxylene adsorption apparatus 13, paraxylene can be concentrated and purified by repeating adsorption and desorption of this mixture using zeolite.

On the other hand, of the benzene and toluene separated as low-boiling point components in the benzene/toluene tower 11, toluene can be converted to benzene in a dealkylation apparatus 14 or converted to benzene and xylenes in a disproportionation (transalkylation) apparatus 15. The heavy aromatics separated and removed from the xylene distillation tower 12 as high-boiling point components can be used as additives for high-octane gasoline, but trimethylbenzene is separated in a heavy aromatic distillation tower 16 and mixed with toluene to carry out disproportionation treatment, whereby a part thereof can be converted to a xylene mixture containing paraxylene and then returned to the inlet side of the purification step (benzene/toluene tower 11, etc.).

Further, if necessary, xylene may be subjected to isomerization treatment. Ortho-xylene, meta-xylene, and ethylbenzene remaining after obtaining high-purity paraxylene in the paraxylene adsorption apparatus 13 can be subjected to isomerization treatment in an isomerization treatment apparatus 17 to partially convert them to paraxylene and then returned to the inlet side of the purification step (xylene tower 12, etc.). Specifically, the isomerization treatment may be carried out by heating a mixture of ortho-xylene, meta-xylene, and ethylbenzene after separating paraxylene and passing it through a reactor packed with a zeolite catalyst.

<Example>

A process flow having the configuration shown in Fig. 1 was assumed. Hydrogen separated by a hydrogen separator 7 (stream 18) and hydrogen generated by an electrolyzer 8 (stream 6) are added to synthesis gas produced in a gasifier 1 (stream 2) to obtain a raw material mixed gas (stream 11), to which gas phase gas (stream 12) returned through the recycle loop is added, and the gas flow (stream 13) heated by a temperature raising device 2 is introduced into a reactor 3 for paraxylene synthesis. The product gas mixture (stream 14) coming out of the reactor 3 is cooled by a temperature lowering device 4, flows into a gas-liquid-liquid separator 5, and is separated into three phases; the gas extracted from the gas phase (stream 12) is returned to the inlet side of the temperature raising device 2 through the recycle loop as described above, but a part thereof is extracted from the recycle loop and divided into hydrogen (stream 18) and purge gas (stream 15) as described above by a hydrogen separator 7. On the other hand, the product liquid extracted from the oil phase (stream 16) is separated and purified into various aromatic compounds including paraxylene in a purification step 6.

Under the above assumptions, the flow rate and composition of each stream were simulated, and the results are shown in Table 1. As shown in Table 1, looking at the raw material mixed gas (stream 11), the mass fraction of hydrogen (H2) is 0.105 (molar fraction $N_{H2}$ = 0.662), the mass fraction of carbon monoxide (CO) is 0.470 (molar fraction $N_{CO}$ = 0.213), and the mass fraction of carbon dioxide (CO2) is 0.417 (molar fraction $N_{CO2}$ = 0.121), so that the R value is 1.62, and the production amount of paraxylene at this time was 2.51 tons/hour.

[Table 1]

| Example: Gasification Gas + Renewable Energy Hydrogen + Recycle PSA Feed | | | | | | | | | | | | | | | |
|---|---|---|---|---|---|---|---|---|---|---|---|---|---|---|---|
| Stream | | 1 | 2 | 3 | 4 | 5 | 6 | 11 | 12 | 13 | 14 | 15 | 16 | 17 | 18 |
| Phase | | Solid | Gas | Gas | Liquid | Gas | Gas | Gas | Gas | Gas | Gas | Gas | Liquid | Liquid | Gas |
| | | | | | | | | | | | | | | | |
| Temperature | C | 20 | 40 | 20 | 20 | 40 | 40 | 40 | 40 | 300 | 361 | 40 | 40 | 40 | 40 |
| Pressure | KPAG | 0 | 4300 | 0 | 0 | 0 | 4300 | 4300 | 3750 | 4200 | 4000 | 50 | 3900 | 3900 | 3750 |
| Molecular Weight | | | 19.1 | 32.0 | 18.0 | 32.0 | 2.0 | 12.7 | 20.8 | 19.4 | 21.1 | 28.9 | 93.1 | 18.1 | 2.0 |
| Total Mass Flow Rate | ton/h | 9 | 17 | 0.3 | 9 | 8 | 1 | 18 | 147 | 165 | 165 | 3 | 5 | 10 | 0.09 |
| Total Molar Flow Rate | kgmol/h | | 887 | 11 | 490 | 245 | 490 | 1419 | 7095 | 8514 | 7841 | 97 | 55 | 552 | 42 |
| | | | | | | | | | | | | | | | |
| Mass Fraction | | | | | | | | | | | | | | | |
| $H_2$ | | | 0.048 | 0.000 | 0.000 | 0.000 | 1.000 | 0.105 | 0.037 | 0.044 | 0.033 | 0.008 | 0.000 | 0.000 | 1.000 |
| CO | | | 0.499 | 0.000 | 0.000 | 0.000 | 0.000 | 0.470 | 0.053 | 0.098 | 0.048 | 0.054 | 0.001 | 0.000 | 0.000 |
| $CO_2$ | | | 0.443 | 0.000 | 0.000 | 0.000 | 0.000 | 0.417 | 0.594 | 0.575 | 0.542 | 0.612 | 0.043 | 0.010 | 0.000 |
| $O_2$ | | | 0.000 | 1.000 | 0.000 | 1.000 | 0.000 | 0.000 | 0.000 | 0.000 | 0.000 | 0.000 | 0.000 | 0.000 | 0.000 |
| $H_2O$ | | | 0.004 | 0.000 | 1.000 | 0.000 | 0.000 | 0.004 | 0.002 | 0.002 | 0.062 | 0.002 | 0.001 | 0.990 | 0.000 |
| $CH_4$ | | | 0.000 | 0.000 | 0.000 | 0.000 | 0.000 | 0.000 | 0.181 | 0.161 | 0.164 | 0.186 | 0.005 | 0.000 | 0.000 |
| C2-C4 Paraffin | | | 0.000 | 0.000 | 0.000 | 0.000 | 0.000 | 0.000 | 0.094 | 0.083 | 0.086 | 0.097 | 0.042 | 0.000 | 0.000 |
| C2-C4 Olefin | | | 0.000 | 0.000 | 0.000 | 0.000 | 0.000 | 0.000 | 0.002 | 0.002 | 0.002 | 0.002 | 0.001 | 0.000 | 0.000 |
| C5+ | | | 0.000 | 0.000 | 0.000 | 0.000 | 0.000 | 0.000 | 0.004 | 0.003 | 0.004 | 0.004 | 0.012 | 0.000 | 0.000 |
| Benzene | | | 0.000 | 0.000 | 0.000 | 0.000 | 0.000 | 0.000 | 0.000 | 0.000 | 0.000 | 0.000 | 0.003 | 0.000 | 0.000 |
| Toluene | | | 0.000 | 0.000 | 0.000 | 0.000 | 0.000 | 0.000 | 0.001 | 0.001 | 0.003 | 0.001 | 0.055 | 0.000 | 0.000 |
| Ethylbenzene | | | 0.000 | 0.000 | 0.000 | 0.000 | 0.000 | 0.000 | 0.000 | 0.000 | 0.000 | 0.000 | 0.010 | 0.000 | 0.000 |
| o-Xylene | | | 0.000 | 0.000 | 0.000 | 0.000 | 0.000 | 0.000 | 0.000 | 0.000 | 0.001 | 0.000 | 0.032 | 0.000 | 0.000 |
| m-Xylene | | | 0.000 | 0.000 | 0.000 | 0.000 | 0.000 | 0.000 | 0.000 | 0.000 | 0.000 | 0.000 | 0.012 | 0.000 | 0.000 |
| p-Xylene | | | 0.000 | 0.000 | 0.000 | 0.000 | 0.000 | 0.000 | 0.003 | 0.003 | 0.018 | 0.004 | 0.489 | 0.000 | 0.000 |

EP 4 455 113 A1

(continued)

| Example: Gasification Gas + Renewable Energy Hydrogen + Recycle PSA Feed | | | | | | | | | | | | | | |
|---|---|---|---|---|---|---|---|---|---|---|---|---|---|---|
| C9 Aromatics | | 0.000 | 0.000 | 0.000 | 0.000 | 0.000 | 0.000 | 0.001 | 0.000 | 0.008 | 0.001 | 0.255 | 0.000 | 0.000 |
| C10 Aromatics | | 0.000 | 0.000 | 0.000 | 0.000 | 0.000 | 0.000 | 0.000 | 0.000 | 0.001 | 0.000 | 0.039 | 0.000 | 0.000 |
| PX Production Amount | | | | | | | | | | | ton/h | 2.51 | | |

<Comparative Example>

The same process flow as in Example shown in Fig. 1 was assumed, and the temperature and pressure of each stream were also assumed to be the same as in Example. However, there was no separation of hydrogen in the hydrogen separator 7 (stream 18) and no generation of hydrogen in the electrolyzer 8 (streams 4, 5, and 6), that is, stream 2 and stream 11 were the same.

Under the above assumptions, the flow rate and composition of each stream were simulated, and the results are shown in Table 2. As shown in Table 2, looking at the raw material mixed gas (stream 11), the weight fraction of hydrogen (H2) is 0.048 (molar fraction $N_{H2}$ = 0.445), the weight fraction of carbon monoxide (CO) is 0.499 (molar fraction $N_{CO}$ = 0.341), and the weight fraction of carbon dioxide (CO2) is 0.443 (molar fraction $N_{CO2}$ = 0.193), so that the R value is 0.49, and the production amount of paraxylene at this time was 2.07 tons/hour.

[Table 2]

| Comparative Example: Gasification Gas-Only Feed | | | | | | | | | | |
|---|---|---|---|---|---|---|---|---|---|---|
| Stream | | 1 | 3 | 11 | 12 | 13 | 14 | 15 | 16 | 17 |
| Phase | | Solid | Gas | Gas | Gas | Gas | Gas | Gas | Liquid | Liquid |
| | | | | | | | | | | |
| Temperature | C | 20 | 20 | 40 | 52 | 300 | 368 | 40 | 40 | 40 |
| Pressure | KPAG | 0 | 0 | 4300 | 4300 | 4200 | 4000 | 50 | 3900 | 3900 |
| Molecular Weight | | | 32.0 | 19.1 | 33.2 | 30.8 | 32.9 | 33.2 | 94.4 | 18.2 |
| Total Mass Flow Rate | ton/h | 14 | 12 | 27 | 235 | 262 | 262 | 16 | 5 | 6 |
| Total Molar Flow Rate | kgmol/h | | 375 | 1419 | 7095 | 8514 | 7962 | 509 | 49 | 309 |
| | | | | | | | | | | |
| Mass Fraction | | | | | | | | | | |
| $H_2$ | | | 0.000 | 0.048 | 0.011 | 0.015 | 0.010 | 0.011 | 0.000 | 0.000 |
| CO | | | 0.000 | 0.499 | 0.135 | 0.173 | 0.130 | 0.135 | 0.002 | 0.000 |
| $CO_2$ | | | 0.000 | 0.443 | 0.811 | 0.773 | 0.782 | 0.811 | 0.092 | 0.021 |
| $O_2$ | | | 1.000 | 0.000 | 0.000 | 0.000 | 0.000 | 0.000 | 0.000 | 0.000 |
| $H_2O$ | | | 0.000 | 0.004 | 0.001 | 0.002 | 0.022 | 0.001 | 0.001 | 0.979 |
| $CH_4$ | | | 0.000 | 0.000 | 0.016 | 0.014 | 0.015 | 0.016 | 0.001 | 0.000 |
| C2-C4 Paraffin | | | 0.000 | 0.000 | 0.011 | 0.010 | 0.011 | 0.011 | 0.008 | 0.000 |
| C2-C4 Olefin | | | 0.000 | 0.000 | 0.001 | 0.001 | 0.001 | 0.001 | 0.001 | 0.000 |
| C5+ | | | 0.000 | 0.000 | 0.002 | 0.001 | 0.002 | 0.002 | 0.007 | 0.000 |
| Benzene | | | 0.000 | 0.000 | 0.000 | 0.000 | 0.000 | 0.000 | 0.003 | 0.000 |
| Toluene | | | 0.000 | 0.000 | 0.001 | 0.000 | 0.001 | 0.001 | 0.040 | 0.000 |
| Ethylbenzene | | | 0.000 | 0.000 | 0.000 | 0.000 | 0.000 | 0.000 | 0.010 | 0.000 |
| o-Xylene | | | 0.000 | 0.000 | 0.000 | 0.000 | 0.001 | 0.000 | 0.032 | 0.000 |
| m-Xylene | | | 0.000 | 0.000 | 0.000 | 0.000 | 0.000 | 0.000 | 0.012 | 0.000 |
| p-Xylene | | | 0.000 | 0.000 | 0.002 | 0.002 | 0.010 | 0.002 | 0.448 | 0.000 |
| C9 Aromatics | | | 0.000 | 0.000 | 0.000 | 0.000 | 0.005 | 0.000 | 0.267 | 0.000 |
| C10 Aromatics | | | 0.000 | 0.000 | 0.000 | 0.000 | 0.001 | 0.000 | 0.076 | 0.000 |
| | | | | | | PX Production Amount | | ton/h | 2.07 | |

EP 4 455 113 A1

<Comparison between Example and Comparative Example>

Comparing the results of Example and Comparative Example described above (shown in Tables 1 and 2), it can be seen that the production amount of paraxylene in Example with an R value of 1.62 is about 21% larger than that in Comparative Example with an R value of 0.49.

<Sensitivity Analysis>

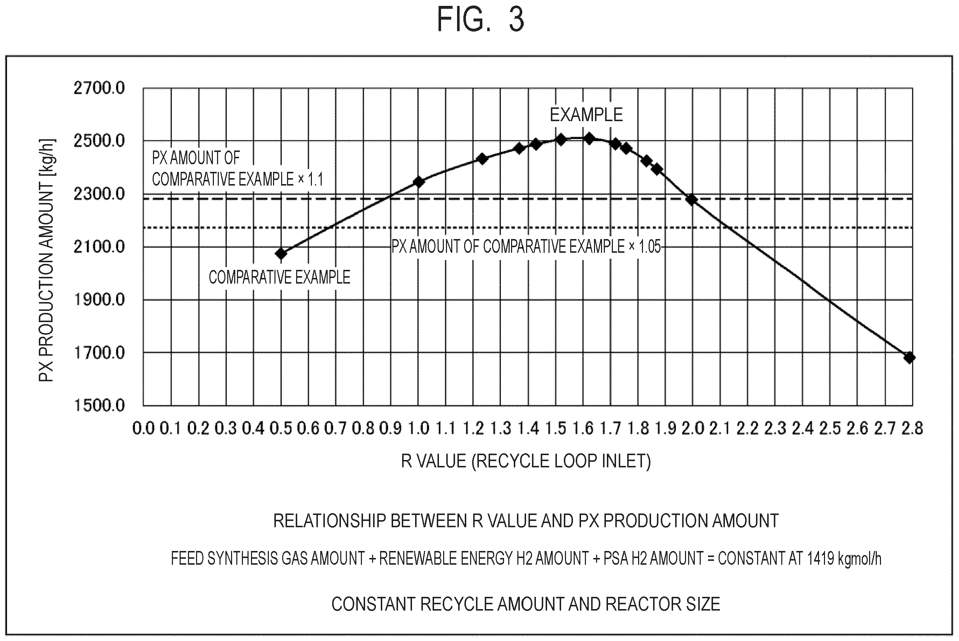

The volumetric (molar) flow rate ratio of the raw material mixed gas (stream 11) to the gas merged from the recycle loop (stream 12) was set to 1:5, other operating conditions and catalyst activity were kept constant, and the relationship between the PX production amount when the R value was changed by changing the amount of hydrogen added to the synthesis gas was simulated; the results are shown in Table 3 and Fig. 3. As can be seen from Table 3 and Fig. 3, the paraxylene (PX) production amount is maximized when the R value is 1.625, and as the R value deviates from 1.625, the paraxylene production amount decreases.

[Table 3]

| Sensitivity Analysis of R Value and PX Production Amount | | | | |
|---|---|---|---|---|
| R Value (Reactor IN) | R Value (Recycle Loop IN (SG + PSA H2 + Renewable Energy H2)) | PX Production Amount | Ratio to Maximum PX Production Amount | |
| - | | kg/h | | |
| -0.436 | 0.500 | 2071.5 | 82.5% | * Comparative Example |
| -0.317 | 1.002 | 2341.2 | 93.3% | |
| -0.199 | 1.236 | 2429.5 | 96.8% | |
| -0.078 | 1.370 | 2470.8 | 98.4% | |
| 0.001 | 1.428 | 2484.4 | 99.0% | |
| 0.195 | 1.520 | 2505.1 | 99.8% | |
| 0.534 | 1.625 | 2510.5 | 100.0% | * Example |
| 1.106 | 1.722 | 2488.4 | 99.1% | |
| 1.380 | 1.758 | 2472.2 | 98.5% | |
| 1.953 | 1.834 | 2426.5 | 96.7% | |
| 2.263 | 1.873 | 2391.3 | 95.3% | |
| 3.267 | 1.997 | 2278.3 | 90.7% | |
| 7.947 | 2.788 | 1684.5 | 67.1% | |

Further, from Fig. 3, it can also be seen that when the paraxylene production amount in the case of not adding hydrogen to the synthesis gas (in Comparative Example) is taken as 1, the range of the R value at which the paraxylene production amount becomes 1.05 or more (5% increase) is 0.7 or more and 2.1 or less, and the range of the R value at which the paraxylene production amount becomes 1.1 or more (10% increase) is 0.9 or more and 2.0 or less.

This application claims priority from Japanese

Patent Application No. 2021-209637 filed on December 23, 2021, the contents of which are incorporated herein by reference.

**Reference Signs List**

1 gasifier
2 temperature raising device
3 reactor
4 temperature lowering device
5 gas-liquid-liquid separator
6 purification apparatus
7 hydrogen separator
8 electrolyzer
11 benzene/toluene tower
12 xylene distillation tower
13 paraxylene adsorption apparatus
14 dealkylation apparatus
15 disproportionation apparatus
16 heavy aromatic distillation tower
17 xylene isomerization apparatus

## Claims

1. A method for producing paraxylene from synthesis gas obtained by gasifying carbon-containing raw materials, comprising a mixing step of obtaining a raw material mixed gas by adding hydrogen to the synthesis gas; a reaction step of obtaining a mixed gas product containing paraxylene by introducing the raw material mixed gas into a reactor and bringing it into contact with a paraxylene synthesis catalyst under heating and pressurization; a separation step of separating the mixed gas product into a gas phase composed of non-condensable components, an oil phase containing paraxylene, and an aqueous phase containing water-soluble components by cooling the mixed gas product to condense high-boiling point components; and a purification step of purifying the oil phase to obtain paraxylene,

   wherein the mixing step adds hydrogen so that the obtained raw material mixed gas has a composition in which an R value defined by the following formula (1) [Math. 1]

$$R = \frac{N_{H2} - N_{CO2}}{N_{CO} + N_{CO2}} \qquad (1)$$

   (where $N_{H2}$ represents a molar fraction of hydrogen, $N_{CO}$ represents a molar fraction of carbon monoxide, and $N_{CO2}$ represents a molar fraction of carbon dioxide) falls within a range of 0.7 or more and 2.1 or less.

2. The method according to claim 1, wherein at least a part of the gas phase separated in the separation step is recycled to the reactor, and hydrogen is separated from the gas purged from the recycle loop and used for at least a part of the hydrogen to be added to the synthesis gas.

3. The method according to claim 2, wherein pressure swing adsorption or membrane separation is carried out to separate the hydrogen.

4. The method according to any one of claims 1 to 3, wherein hydrogen obtained by electrolyzing water using electric power derived from renewable energy is used for at least a part of the hydrogen to be added to the synthesis gas.

5. The method according to claim 4, wherein oxygen by-produced when electrolyzing the water is used for gasifying the carbon-containing raw materials.

6. The method according to any one of claims 1 to 5, wherein reaction conditions in the reaction step are such that an outlet-side reaction temperature is 250°C or more and 600°C or less, and a reaction pressure is 1 MPaG or more and 10 MPaG.

7. The method according to any one of claims 1 to 6, wherein a catalyst including a first catalyst containing at least one metal oxide selected from chromium, zinc, and copper and a second catalyst containing ZSM-5 zeolite is used in the reaction step.

# FIG. 1

EP 4 455 113 A1

# FIG. 2

EP 4 455 113 A1

# FIG. 3

RELATIONSHIP BETWEEN R VALUE AND PX PRODUCTION AMOUNT

FEED SYNTHESIS GAS AMOUNT + RENEWABLE ENERGY H2 AMOUNT + PSA H2 AMOUNT = CONSTANT AT 1419 kgmol/h

CONSTANT RECYCLE AMOUNT AND REACTOR SIZE

## INTERNATIONAL SEARCH REPORT

International application No.

**PCT/JP2022/047306**

**A.    CLASSIFICATION OF SUBJECT MATTER**

*C07C 1/04*(2006.01)i; *C07C 1/24*(2006.01)i; *C07C 15/08*(2006.01)i; *C07C 29/151*(2006.01)i; *C07C 31/04*(2006.01)i; *C07B 61/00*(2006.01)n

FI:    C07C1/04; C07C15/08; C07C31/04; C07C29/151; C07C1/24; C07B61/00 300

According to International Patent Classification (IPC) or to both national classification and IPC

**B.    FIELDS SEARCHED**

Minimum documentation searched (classification system followed by classification symbols)

C07C1/04; C07C1/24; C07C15/08; C07C29/151; C07C31/04; C07B61/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | JP 2019-205969 A (NIPPON STEEL CORP) 05 December 2019 (2019-12-05) claims, examples, etc. | 1-7 |
| A | US 2021/0179513 A1 (IFP ENERGIES NOUVELLES) 17 June 2021 (2021-06-17) claims, examples, etc. | 1-7 |
| A | JP 2005-15286 A (JAPAN STEEL WORKS LTD) 20 January 2005 (2005-01-20) claims, examples, etc. | 1-7 |
| A | JP 58-192833 A (SHINNENRIYOUYU KAIHATSU GIJUTSU KENKIYUU KUMIAI) 10 November 1983 (1983-11-10) claims, examples, etc. | 1-7 |
| A | JP 2012-520384 A (SAUDI BASIC IND CORP) 06 September 2012 (2012-09-06) claims, examples, etc. | 1-7 |
| A | JP 2013-504651 A (UNIV MASSACHUSETTS) 07 February 2013 (2013-02-07) claims, examples, etc. | 1-7 |

☑ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **06 March 2023** | **20 March 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/047306**

C.      DOCUMENTS CONSIDERED TO BE RELEVANT

| Category* | Citation of document, with indication, where appropriate, of the relevant passages | Relevant to claim No. |
|---|---|---|
| P, A | JP 2022-171430 A (CHIYODA CORP) 11 November 2022 (2022-11-11) claims, examples, etc. | 1-7 |

Form PCT/ISA/210 (second sheet) (January 2015)

## INTERNATIONAL SEARCH REPORT
### Information on patent family members

International application No.

**PCT/JP2022/047306**

| Patent document cited in search report | | | Publication date (day/month/year) | Patent family member(s) | | | Publication date (day/month/year) |
|---|---|---|---|---|---|---|---|
| JP | 2019-205969 | A | 05 December 2019 | (Family: none) | | | |
| US | 2021/0179513 | A1 | 17 June 2021 | FR claims, examples CN | 3104576 112979403 | A A | |
| JP | 2005-15286 | A | 20 January 2005 | (Family: none) | | | |
| JP | 58-192833 | A | 10 November 1983 | (Family: none) | | | |
| JP | 2012-520384 | A | 06 September 2012 | US claims, examples WO CN | 2012/0123001 2010/105786 102356044 | A1 A1 A | |
| JP | 2013-504651 | A | 07 February 2013 | US claims, examples, etc. WO CN KR | 2012/0203042 2011/031320 102666796 10-2012-0104520 | A1 A2 A A | |
| JP | 2022-171430 | A | 11 November 2022 | WO claims, examples | 2022/230467 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020535966 W
- JP 2019205969 A
- JP 2010070763 A
- US 20080098654
- US 2021209637 A

**Non-patent literature cited in the description**

- **PEIPEI ZHANG et al.** Chemical Science. The Royal Society of Chemistry, October 2017, vol. 8, 7941-7946